(19) Europäisches Patentamt
European Patent Office
Office européen des brevets

(11) Veröffentlichungsnummer : **0 151 249**
**B1**

(12) # EUROPÄISCHE PATENTSCHRIFT

(45) Veröffentlichungstag der Patentschrift : **30.09.87**

(51) Int. Cl.⁴ : **C 01 B 21/14**, C 07 C 51/42

(21) Anmeldenummer : **84114251.6**

(22) Anmeldetag : **26.11.84**

(54) **Stabilisierte Lösungen von Hydroxylamin oder dessen Salzen in Wasser oder Alkoholen sowie deren Herstellung.**

(30) Priorität : **02.12.83 DE 3343597**

(43) Veröffentlichungstag der Anmeldung : **14.08.85 Patentblatt 85/33**

(45) Bekanntmachung des Hinweises auf die Patenterteilung : **30.09.87 Patentblatt 87/40**

(84) Benannte Vertragsstaaten : **BE CH DE FR GB IT LI NL**

(56) Entgegenhaltungen :
**US-A- 3 145 082**
**US-A- 3 480 391**
**US-A- 3 480 392**
**US-A- 3 544 270**

(73) Patentinhaber : **BASF Aktiengesellschaft**
**Carl-Bosch-Strasse 38**
**D-6700 Ludwigshafen (DE)**

(72) Erfinder : **Grosskinsky, Otto-Alfred, Dr.-Chem.**
**Semmelweisstrasse 8**
**D-6700 Ludwigshafen (DE)**
Erfinder : **Frommer, Elmar, Dr.-Chem.**
**Lisztstrasse 117**
**D-6700 Ludwigshafen (DE)**
Erfinder : **Ritz, Josef, Dr.-Chem.**
**Osloer Weg 8**
**D-6700 Ludwigshafen (DE)**
Erfinder : **Thomas, Erwin**
**Borngasse 12**
**D-6713 Freinsheim (DE)**
Erfinder : **Weiss, Franz-Josef, Dr.-Chem.**
**Schilfweg 1**
**D-6708 Neuhofen (DE)**

**EP 0 151 249 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Lösungen von Hydroxylammoniumsalzen zersetzen sich langsam bei Raumtemperatur und schneller bei erhöhter Temperatur. Dies gilt in vermehrtem Maße für Lösungen von freiem Hydroxylamin. Es hat nicht an Versuchen gefehlt, Lösungen von Hydroxylamin und dessen Salze zu stabilisieren, um eine verbesserte Lagerfähigkeit zu erzielen. So verwendet man als Stabilisierungsmittel Harnstoffderivate entsprechend der US-A-3 544 270, Amidoxime entsprechend der US-A-3 480 391 oder Hydroxamsäuren entsprechend der US-A-3 480 392. Aus der US-A-3 145 082 ist auch schon bekannt, chelatbildente Mittel wie das Natriumsalz der Ethylendiamintetraessigsäure als Stabilisierungsmittel zu verwenden. Die bislang verwendeten Stabilisierungsmittel sind noch verbesserungsbedürftig.

Es war die technische Aufgabe gestellt, stabilisierte Lösungen von Hydroxylamin oder dessen Salzen zur Verfügung zu stellen, die über einen längeren Zeitraum stabil sind und insbesondere die Zersetzung von freiem Hydroxylamin minimiert wird.

Diese Aufgabe wird gelöst durch stabilisierte Lösungen von Hydroxylamin oder dessen Salzen in Wasser oder Alkoholen, gekennzeichnet durch einen Gehalt an Flavonen der Formel

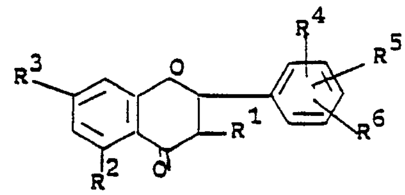

in der $R^1$ bis $R^6$ jeweils ein Wasserstoffatom, eine Hydroxylgruppe, eine $C_1$- bis $C_4$-Alkoxygruppe oder eine $C_1$- bis $C_4$-Acetoxygruppe bezeichnet, mit der Maßgabe, daß mindestens ein Substituent eine Hydroxylgruppe ist.

Ferner ist ein Gegenstand der Erfindung ein Verfahren zur Herstellung von stabilisierten Lösungen von Hydroxylamin oder dessen Salzen durch Zugabe von Stabilisatoren, dadurch gekennzeichnet, daß man aus der zu stabilisierenden Lösung den darin gelösten molekularen Sauerstoff durch Behandeln mit Stickstoff, der frei von molekularem Sauerstoff ist, entfernt und dann Flavone, die mindestens eine Hydroxylgruppe enthalten, zusetzt.

Die gemäß der Erfindung stabilisierten Lösungen von Hydroxylamin oder dessen Salzen haben den Vorteil, daß sie über längere Zeit als bisher stabil sind und insbesondere die Zersetzung von freiem Hydroxylamin auf ein Minimum reduziert wird.

Erfindungsgemäß geht man von Lösungen von Hydroxylamin oder dessen Salzen in Wasser oder Alkoholen, z. B. $C_1$- bis $C_4$-Alkanolen aus. Geeignete Salzen des Hydroxylamins sind beispielsweise solche mit starken Mineralsäuren, wie Schwefelsäure, Salpetersäure, Chlorwasserstoffsäure oder solche mit Fettsäuren, z. B. Essigsäure oder Propionsäure. Aufgrund der verschiedenen Löslichkeiten liegt Hydroxylamin vorzugsweise gelöst in Wasser oder Alkoholen vor, während dessen Salze vorzugsweise als wäßrige Lösungen vorliegen. Der Gehalt an Hydroxylamin oder dessen Salzen beträgt in der Regel von 10 bis 70 Gew.%. Die Ausgangslösungen haben in der Regel einen pH-Wert von 8 bis 11. Besonders bevorzugt geht man von wäßrigen Hydroxylaminlösungen aus.

Als Stabilisatoren verwendet man Flavone der Formel

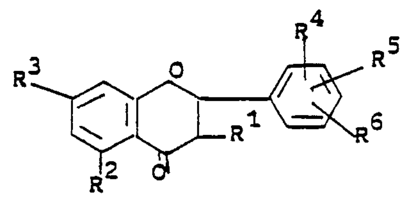

in der $R^1$ bis $R^6$ jeweils ein Wasserstoffatom, eine Hydroxlgruppen, eine $C_1$- bis $C_4$-Alkoxygruppe oder eine $C_1$- bis $C_4$-Acetoxygruppe bezeichnet, mit der Maßgabe, daß mindestens ein Substituent eine Hydroxylgruppe ist. In besonders bevorzugten Flavonen der Formel I bezeichnen $R^1$ bis $R^5$ jeweils ein Wasserstoffatom, eine Hydroxylgruppe oder eine Methoxygruppe, mit der Maßgabe, daß mindestens drei Substituenten eine Hydroxylgruppe bedeuten. Die Hydroxylgruppen enthaltenden Flavone können auch als Glykoside vorliegen.

Geeignete Flavone sind beispielsweise :

3,5,7,2',4'-Penta-hydroxy-flavon (Morin)
3,5,7,3',3'-Penta-hydroxy-flavon (Quercetin)
3,5,7,3',4',5'-Hexa-hydroxy-flavon (Myricetin)

3,5,7,8',3',4'-Hexa-hydroxy-flavon (Gossypetin)
3,7,3',4',5'-Penta-hydroxy-flavon (Robinetin)
3,5,7,4'-Tetra-hydroxy-flavon (Kämpferol)
5,7,3',4'-Tetra-hydroxy-flavon (Luteolin)
3,7,3',4'-Tetra-hydroxy-flavon (Fisetin)
5,7,4'-Tri-hydroxy-flavon (Apigenin)
3,5,7-Tri-hydroxy-flavon (Galgangin)
5,7-Di-hydroxy-flavon (Chrysin) und
3-Mono-hydroxy-flavon (Flavonol).

Vorteilhaft wendet man die Hydroxylgruppen enthaltenden Flavone in Mengen von 0,005 bis 1 Gew.%, insbesondere 0,01 bis 0,1 Gew.%, bezogen auf die zu stabilisierende Lösung an. Es hat sich ferner bewährt, wenn man zusätzlich Polyhydroxybenzole, insbesondere Pyrogallol mitverwendet. Polyhydroxybenzole werden vorteilhaft in Mengen von 0,005 bis 0,1 Gew.%, bezogen auf die zu stabilisierende Lösung zugesetzt. Es ist bemerkenswert, daß durch die kombinierte Anwendung von Hydroxylgruppen enthaltenden Flavonen und Polyhydroxybenzolen eine synergistische Wirkung erzielt wird.

Stabilisierte Lösungen von Hydroxylamin oder dessen Salzen in Wasser oder Alkoholen werden erfindungsgemäß hergestellt, indem man aus den zu stabilisierenden Lösungen zunächst den darin gelöst enthaltenen molekularen Sauerstoff durch Behandeln mit Stickstoff, der frei von molekularem Sauerstoff ist, verdrängt. Dies erzielt man beispielsweise dadurch daß man durch die zu stabilisierende Lösung sauerstoffreien Stickstoff leitet, z. B. für 5 bis 10 Minuten. Dann gibt man Hydroxylgruppen enthaltende Flavone und ggf. Polyhydroxybenzole zu und löst diese in der zu stabilisierenden Lösung auf. Es ist auch möglich, die Stabilisatoren bereits in gelöstem Zustand den zu stabilisierenden Lösungen zuzufügen.

Die erfindungsgemäßen Zusätze stabilisieren wäßrige Hydroxylaminlösungen vorteilhaft im Bereich von + 5 bis 40 °C.

Es versteht sich, daß es von Vorteil ist, Kontamination von zu stabilisierenden Lösungen mit Schwermetallen, insbesondere Kupfer oder Edelmetallen zu vermeiden, da diese die Zersetzung von Hydroxylamin katalysieren. Ferner ist es von Vorteil, energiereiche Strahlung durch geeignet eingefärbte Glasbehälter auszuschalten. Ferner ist es von Vorteil, die stabilisierten Lösungen bei Temperaturen von < 40 °C, z. B. bei Temperaturen von 5 bis 20 °C aufzubewahren.

Stabilisierte Lösungen von Hydroxylamin oder dessen Salze eignen sich zur Herstellung von Oximen.

Der erfindungsgemäße Gegenstand sei in folgenden Beispielen veranschaulicht.

## Beispiel 1 und Vergleichsbeispiele 1 und 2

Eine wäßrige Lösung von ca. 10 Gew.% Hydroxylamin wird bei 20 °C 10 Minuten mit sauerstoffreiem Stickstoff begast und dann die Stabilisatoren zugefügt. Die Konzentration an Hydroxylamin, die zugegebene Art und Menge an Stabilisatoren sowie die in Abhängigkeit der Zeit erzielten Ergebnisse sind aus Tabelle 1 ersichtlich :

Tabelle 1

| | Stabilisator | Zeit [Stunden] 0 | 20 | 112 | 287 | 668 |
|---|---|---|---|---|---|---|
| Beispiel 1 | Morin 0,02 Gew.% | 100,5 | 100,15 | - | 99,66 | 93,33 g/l $NH_2OH$ |
| Vergleichs-beispiel 1 | - | 138,5 | 134,6 | - | 105,6 | 76,6 g/l $NH_2OH$ |
| Vergleichs-beispiel 2 | Pyrogallol 0,005 Gew.% | 101,0 | 99,9 | 97,0 | 96,2 | 94,5 g/l $NH_2OH$ |

## Beispiele 2 bis 4

Man verfährt wie in Beispiel 1 beschrieben. Die Konzentration an Hydroxylamin, die zugegebene Art und Menge an Stabilisatoren sowie die in Abhängigkeit von Zeit und Temperatur erzielten Ergebnisse sind aus Tabelle 2 zu entnehmen.

Tabelle 2

| Beispiel | Stabilisator | °C | | | | |
|---|---|---|---|---|---|---|
| | | | 0 | 743 | 1196 | Stunden |
| 2 | 50 ppm Morin + 50 ppm Pyrogallol | 5 | 110,55 | 110,48 | 110,06 | g/l $NH_2OH$ |
| | | | 0 | 404 | 1195 | Stunden |
| | " | 20 | 110,25 | 110,06 | 109,89 | g/l $NH_2OH$ |
| | | | 0 | 359 | 1194 | Stunden |
| | " | 40 | 110,25 | 109,73 | 108,08 | g/l $NH_2OH$ |
| 3 | 50 ppm Quercetin | | 0 | 384 | 526 | Stunden |
| | | 5 | 136,95 | 136,62 | 136,46 | g/l $NH_2OH$ |
| | | | 0 | 384 | 523 | Stunden |
| | " | 20 | 136,95 | 135,96 | 135,63 | g/l $NH_2OH$ |
| | | | 0 | 384 | 522 | Stunden |
| | " | 40 | 136,95 | 135,96 | 135,63 | g/l $NH_2OH$ |
| | | | 0 | 162 | 1656 | Stunden |
| 4 | Morin 25 mg/mol $NH_2OH$ | 25 | 473,55 | 454,08 | 414,15 | g/l $NH_2OH$ |

**Patentansprüche**

1. Stabilisierte Lösungen von Hydroxylamin oder dessen Salzen in Wasser oder Alkoholen, gekennzeichnet durch einen Gehalt an Flavonen der Formel

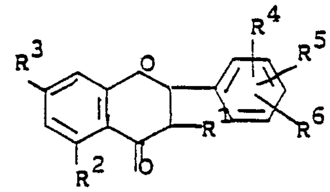

(I)

in der $R^1$ bis $R^6$ jeweils ein Wasserstoffatom, eine Hydroxylgruppe, eine $C_1$- bis $C_4$-Alkoxygruppe oder eine $C_1$- bis $C_4$-Acetoxygruppe bezeichnen, mit der Maßgabe, daß mindestens ein Substituent eine Hydroxylgruppe ist.

2. Stabilisierte Lösungen nach Anspruch 1, dadurch gekennzeichnet, daß in den Flavonen der Formel (I) $R^1$ bis $R^6$ jeweils ein Wasserstoffatom, eine Hydroxylgruppe oder eine Methoxygruppe bezeichnen, mit der Maßgabe, daß mindestens drei Substituenten eine Hydroxylgruppe bedeuten.

3. Stabilisierte Lösungen nach Anspruch 1, gekennzeichnet durch einen Gehalt an Morin.

4. Stabilisierte Lösungen nach Anspruch 1, gekennzeichnet durch einen Gehalt von 0,005 bis 1 Gew.% Flavonen der Formel I, bezogen auf die zu stabilisierende Lösung.

5. Stabilisierte Lösungen nach Anspruch 1, gekennzeichnet durch einen zusätzlichen Gehalt an Pyrogallol.

6. Verfahren zur Herstellung von stabilisierten Lösungen von Hydroxylamin oder dessen Slazen in Wasser oder Alkoholen durch Zusatz von Stabilisatoren, dadurch gekennzeichnet, daß man aus den zu stabilisierenden Lösungen den darin gelöst enthaltenen molekularen Sauerstoff durch Behandeln mit Stickstoff, der frei von molekularem Sauerstoff ist, entfernt und dann Flavone der Formel

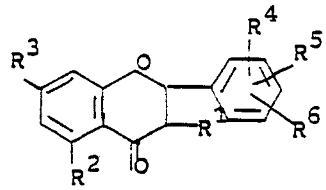

in der $R^1$ bis $R^6$ jeweils ein Wasserstoffatom, eine Hydroxylgruppe, eine $C_1$- bis $C_4$-Alkoxygruppe oder eine $C_1$- bis $C_4$-Acetoxygruppe bezeichnen, mit der Maßgabe, daß mindestens ein Substituent eine Hydroxylgruppe ist, zusetzt.

## Claims

1. A stabilized solution of hydroxylamine or its salts in water or an alcohol, which contains a flavone of the formula

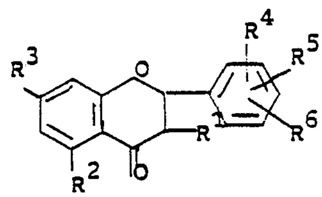

(I)

where $R^1$, $R^2$, $R^3$, $R^4$, $R^5$ and $R^6$ are each hydrogen, hydroxyl, $C_1$-$C_4$-alkoxy or $C_1$-$C_4$-acetoxy, with the proviso that at least one substituent is hydroxyl.

2. A stabilized solution as claimed in claim 1, wherein, in the flavone of the formula (I), $R^1$, $R^2$, $R^3$, $R^4$, $R^5$ and $R^6$ are each hydrogen, hydroxyl or methoxy, with the proviso that at least three substituents are hydroxyl.

3. A stabilized solution as claimed in claim 1, which contains morin.

4. A stabilized solution as claimed in claim 1, which contains from 0.005 to 1 % by weight, based on the solution to be stabilized, of a flavone of the formula (I).

5. A stabilized solution as claimed in claim 1, which additionally contains pyrogallol.

6. A process for the preparation of a stabilized solution of hydroxylamine or its salts in water or an alcohol by adding a stabilizer, wherein the molecular oxygen dissolved in the solution to be stabilized is removed from this solution by treatment with nitrogen which is free of molecular oxygen, and a flavone of the formula

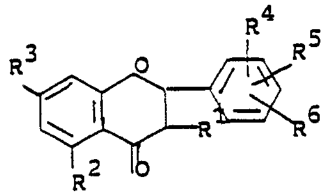

where $R^1$, $R^2$, $R^3$, $R^4$, $R^5$ and $R^6$ are each hydrogen, hydroxyl, $C_1$-$C_4$-alkoxy or $C_1$-$C_4$-acetoxy, with the proviso that at least one substituent is hydroxyl, is then added.

## Revendications

1. Solutions stabilisées d'hydroxylamine ou ses sels dans de l'eau ou des alcools, caractérisées par le fait qu'elles contiennent des flavones de formule

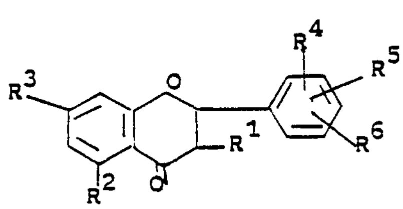

(I)

dans laquelle $R^1$ à $R^6$ représentent chacun un atome d'hydrogène, un groupe hydroxyle un groupe alcoxy en $C_1$ à $C_4$ ou un groupe acétoxy en $C_1$ à $C_4$, sous réserve qu'au moins un substituant soit un groupe hydroxyle.

2. Solutions stabilisées selon la revendication 1, caractérisées par le fait que, dans les flavones de formule (I), $R^1$ à $R^6$ représentent chacun un atome d'hydrogène, un groupe hydroxyle ou un groupe méthoxy, sous réserve qu'au moins un substituant soit un groupe hydroxyle.

3. Solutions stabilisées selon la revendication 1, caractérisées par le fait qu'elles contiennent de la morine.

4. Solutions stabilisées selon la revendication 1, caractérisées par le fait qu'elles contiennent de 0,005 à 1 % en poids de flavones de formule (I), rapportés à la solution à stabiliser.

5. Solutions stabilisées selon la revendication 1, caractérisées par le fait qu'elles contiennent, en outre, du pyrogallol.

6. Procédé de préparation de solutions stabilisées d'hydroxylamine ou ses sels dans de l'eau ou des alcools par addition de stabilisant, caractérisé par le fait que, par traitement avec de l'azote exempt d'oxygène moléculaire, on élimine des solutions à stabiliser l'oxygène moléculaire qui y est contenu dissous puis on ajoute des flavones de formule

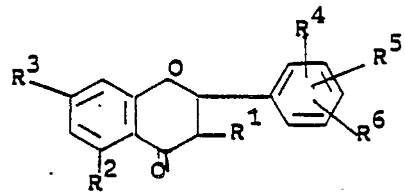

dans laquelle $R^1$ à $R^6$ représentent chacun un atome d'hydrogène, un groupe hydroxyle, un groupe alcoxy en $C_1$ à $C_4$ ou un groupe acétoxy en $C_1$ à $C_4$, sous réserve qu'au moins un substituant soit un groupe hydroxyle.